# EUROPEAN PATENT SPECIFICATION

(11) **EP 2 213 694 B1**
(45) Date of publication and mention of the grant of the patent: **31.07.2013**
(21) Application number: 08829079.6
(22) Date of filing: 02.09.2008
(51) Int. Cl.: C08G 63/06, C08G 63/82, C12P 7/62, C08L 101/16

(54) **12-HYDROXYSTEARIC ACID COPOLYMER AND METHOD FOR PRODUCING THE SAME**
12-HYDROXYSTEARINSÄURE-COPOLYMER UND VERFAHREN ZU SEINER HERSTELLUNG
COPOLYMÈRE D'ACIDE 12-HYDROXYSTÉARIQUE ET SON PROCÉDÉ DE PRODUCTION

(30) Priority: 04.09.2007 JP 2007228753
(43) Date of publication of application: 04.08.2010
(73) Proprietor: Keio University, Tokyo 108-8345 (JP)
(72) Inventor: EBATA, Hiroki, Yokohama-shi Kanagawa 223-8522 (JP); MATSUMURA, Shuichi, Yokohama-shi Kanagawa 223-8522 (JP)
(74) Representative: polypatent BGL
(86) International application number: PCT/JP2008/065753
(87) International publication number: WO 2009/031531

(56) References cited:
- WO-A1-2008/029805
- WO-A2-02/072639
- JP-A- 05 211 878
- JP-A- 10 218 977
- JP-A- 11 012 224
- JP-A- 11 106 488
- JP-A- 2003 012 779
- JP-A- 2005 113 001
- EBATA H, TOSHIMA K, MATSUMURA S: "LIPASE-CATALYZED SYNTHESIS AND PROPERTIES OF POLY[(12-HYDROXYDECANOATE)-CO-(12-HYDROXYS TEARATE)] - DIRECTED TOWARDS NOVEL GREEN AND SUSTAINABLE ELASTOMERS", MACROMOLECULAR BIOSCIENCE, vol. 2008, no. 8, 22 October 2007 (2007-10-22), pages 38-45, XP002674297,
- RAIA SLIVNIAK AND ABRAHAM J.DOMB: 'Lactic acid and ricinoleic acid based copolyesters' MACROMOLECULES vol. 38, no. 13, 28 June 2005, pages 5545 - 5553, XP008131366
- RAIA SLIVNIAK AND ABRAHAM J.DOMB: 'Macrolactones and polyesters from ricinoleic acid' BIOMACROMOLECULES vol. 6, no. 3, June 2005, pages 1679 - 1688, XP008131362

## Description

### Technical Field

The present invention relates to a 12-hydroxystearic acid copolymer which is a new non-petroleum thermoplastic elastomer.
The present invention also relates to a biodegradable thermoplastic elastomer obtained by copolymerizing 12-hydroxystearic acid or derivative thereof, as well as an elastomer composition that contains the same.

### Background Art

Recent years have seen a global escalation in petroleum price stemmed from the intensifying competition to secure petroleum as a material or energy resource, due partly to the straining of political situations in the Middle East including Iraq and Iran and partly to the industrial growth in China and other emerging countries. As a result, there have been active efforts to develop alternative energy sources to replace petroleum, as well as materials based on natural ingredients to replace petroleum materials.
On the other hand, development efforts are also active in the area of energy-saving devices and manufacturing methods thereof for the purpose of suppressing carbon dioxide generations in product manufacturing processes and thereby preventing global warming, and also in the area of recyclable materials such as biodegradable materials and products that can ensure safety in order to prevent environmental pollution.

Among recyclable materials, biodegradable polyesters, especially aliphatic polyesters, are drawing strong attention from the R&D community.
For example, thermoplastic biodegradable aliphatic copolyesters constituted by aliphatic dicarboxylic acid or ester thereof, aliphatic or alicyclic diol, and naturally produced unsaturated acid or ester thereof, are disclosed in Patent Literatures 1 to 3, while a method of manufacturing a biodegradable aliphatic polyester using a tin catalyst from at least one type of aliphatic dicarboxylic acid or ester thereof and at least one type of straight-chain or branched aliphatic glycol is disclosed in Patent Literature 4.
In addition, a biodegradable vegetable oil grease constituted by a natural oil or synthetic triglyceride, at least one of alkyl phenol, benzotriazol and aromatic amine, and alkali metal, alkaline earth metal or other metal-based substance, is disclosed in Patent Literature 5.

Furthermore, Non-patent Literature 1 reports a biodegradable polyester containing ricinoleic acid produced from castor oil, wherein such biodegradable polyester is produced by copolymerization of ricinoleic acid lactone and lactic acid by means of the high-temperature decompression method and ring-opening polymerization via lactone that results in a copolymer with a molecular weight of 5000 to 16000 and melting point of 100 to 130°C. It is also reported that its use in DDS is being examined and the obtained copolymer is less crystalline than polylactic acid and has good biodegradability.
In addition, Non-patent Literature 2 reports an experiment in which polyesters were synthesized by thermal condensation from ricinoleic acid and lactic acid blended at various ratios, followed by transesterification with a polylactic acid of high molecular weight, to achieve random copolymers with a molecular weight of 2000 to 8000. These Non-patent Literatures discuss polyester synthesis based on thermal condensation and thus the obtained polyesters containing ricinoleic acid have a small molecular weight, and the two literatures do not report anything regarding the physical properties or performance of the obtained polyesters copolymerized with lactic acid.
On the other hand, a polyester compound containing the amino group at the end of the molecule, constituted by fatty acid and aliphatic dicarboxylic acid containing the hydroxyl group, is disclosed in Patent Literature 6. Also, a reactive biodegradable copolymer polyester for use as medical material, produced by a polycondensation reaction of ricinoleic acid and lactic acid implemented in a manner controlling the content of ricinoleic acid, is disclosed in Patent Literature 7. However, these substances are both obtained by means of a thermal condensation reaction just like the polyester synthetic method disclosed in Non-patent Literatures 1 and 2.
In addition, Patent Literature 8 discloses a sheet material offering good mechanical strength and elasticity as well as wear resistance and hydrolysis resistance, wherein such sheet material is an elastomer cross-linked with ricinoleic acid ester produced by forming an elastomer in the presence of a peroxide initiator from a polyester which in turn is formed by castor oil or ricinoleic acid ester, epoxidized oil and polycarboxylic acid.

Furthermore, a method of synthesizing a biodegradable polyester using an enzyme, instead of by a condensation reaction using heat, is reported. To be specific, this synthesis method promotes the esterification reaction as part of an equilibrium reaction by using lipase which is a hydrolysis enzyme, wherein an ester is synthesized from oil or fatty acid using an immobilized lipase intended for efficient use of an enzyme lipase.
Reflecting the aforementioned viewpoints, Patent Literature 9 discloses a method of manufacturing an oligomer from ricinoleic acid using an immobilized lipase that has been adsorbed and immobilized onto a carrier constituted by calcined zeolite, while adjusting the water content in the carrier to 800 mg or less per 1 g of immobilized enzyme. In the manufacture of oligomer from ricinoleic acid as disclosed in this literature, an appropriate temperature of the enzyme reaction used in the synthesis is lower than when other thermo-chemical reaction is used, which leads to energy saving and elimination of need for any harmful organic solvent or catalyst and therefore this ester oligomer synthesis method is desirable from the viewpoints of preventing global warming and environmental pollution.
However, the example given in Patent Literature 9 tracks the dehydration ratio based on the neutralization number, and no polyester with a neutralization number of 30 or less was obtained in the example. When estimated from their neutralization numbers, the obtained polyesters likely have a weight-average molecular weight of not exceeding 3000 and therefore these polyesters are deemed relatively small in molecular weight.

Based on the aforementioned published literatures, we cannot argue that technologies are available that can produce, in an industrial setting, those biodegradable polyesters with excellent flexibility that are intended to achieve petroleum independent and do not contribute to global warming or environmental pollution.
On the other hand, thermoplastic elastomers (TPEs) that constitute the field of soft materials to which the present invention belongs, are seeing a rapid growth in use in recent years as recyclable rubber materials. Unlike vulcanized rubbers, TPEs do not require cross-linking of the matrix phase because these materials are designed in such a way that rubber-like elasticity is expressed only by pseudo (physical) cross-linking. TPEs exhibit rubber-like elasticity just like vulcanized rubbers at normal temperature, while at high temperature they can be handled in the same manner as thermoplastic resins because their matrix phase is plasticized and flows. Reversible use (recycling) is also possible, and because no cross-linking process is needed, TPEs allow for substantial energy saving and productivity improvement as compared to vulcanized rubbers. Easy collection and recycling of scraps is another big advantage. Also, reinforcement mechanisms used with TPEs are different from those used with vulcanized rubbers, meaning that TPEs do not require carbon black and other reinforcement materials and thus their specific gravity (weight) remains low.

TPEs comprise a soft segment constituted by soft rubber and a hard segment constituted by hard resin. TPEs are largely classified by their molecular structure into (1) olefin TPEs (TPOs), (2) styrene TPEs (TPSs), (3) urethane TPEs (TPUs), (4) polyester TPEs (TPEEs), (5) polyvinyl chloride TPEs (T-PVCs) and (6) other TPEs.
TPE demand is increasing in the fields of automobiles, construction materials, sporting equipment, medical applications and industrial parts, among others, at such a high rate as approx. 6 to 10% per year. Among others, TPSs and TPOs are seeing a rapid growth in demand as alternative materials to polyvinyl chloride and synthetic rubbers against the background of tightening environmental regulations, and their applications are increasing steadily in a wide range of fields. However, all of these materials are made from petroleum and therefore subject to escalating petroleum price. Another drawback is that they are not biodegradable.

Among others, an ethylene-octene block copolymer, which is a type of TPO, is disclosed in Non-patent Literature 3 as a thermoplastic elastomer made from petroleum material, where it is suggested that the thermal properties and mechanical properties of such block copolymer can be changed in a desired manner by changing the ratio of the hard block containing less octene and soft block containing more octene to be mixed. This block copolymer reportedly offers both flexibility and heat resistance.

Patent Literature 1: Published Japanese Translation of PCT Application No. 2005-523355, which corresponds to WO2003/089491
Patent Literature 2: Published Japanese Translation of PCT Application No. 2005-523356, which corresponds to WO2003/089492
Patent Literature 3: Published Japanese Translation of PCT Application No. 2005-523357, which corresponds to WO2003/089493
Patent Literature 4: Published Japanese Translation of PCT Application No. 2002-539309, which corresponds to WO00/55236
Patent Literature 5: Japanese Patent Laid-open No. Hei 10-46180
Patent Literature 6: Japanese Patent Laid-open No. Hei 5-125166
Patent Literature 7: Japanese Patent Laid-open No. 2005-113001
Patent Literature 8: Published Japanese Translation of PCT Application No. 2006-516998, which corresponds to WO2004/063245
Patent Literature 9: Japanese Patent Laid-open No. Hei 5-211878
Non-patent Literature 1: Biomacromolecules 2005, 6, 1679-1688
Non-patent Literature 2: Macromolecules 2005, 38, 5545-5553
Non-patent Literature 3: Macromolecules 2007, 40, 2852-2862

These and other synthetic rubbers are used in all industrial fields, but stable supply of their material, or petroleum, is at risk. In addition, they are persistent substances and therefore contribute to environmental pollution when disposed of. Furthermore, their polymers contain metal residues. For the environment and living organisms, polymers containing no metal residues are more desirable.

### Summary of the Invention

### Problems to Be Solved by the Invention

In view of the current state described above, synthetic polymer materials made from petroleum and elastomers that use these materials are mainly used in industrial applications, which presents a number of problems from the viewpoints of needs for petroleum independence, prevention of environmental pollution and energy saving. To solve these problems, synthesis of functional polymers offering excellent reaction efficiency and minimal environmental burdens using biocatalysts from alternative material resources to petroleum is required.

### Means for Solving the Problems

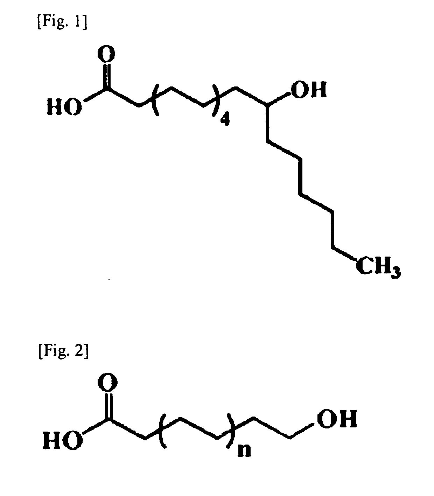

To achieve petroleum independence, energy saving and prevention of environmental pollution, as mentioned above, the inventor found a method of manufacturing a biodegradable elastomer offering excellent flexibility, wherein such method does not use any substances harmful to the human body, but it uses natural materials instead, to which an enzyme or other catalyst present in the natural world is added to trigger a catalytic reaction efficiently in a temperature range lower than what is required in normal chemical reactions.
To be specific, the inventor found a method of synthesizing a biodegradable copolymer polyester offering excellent flexibility useful in industrial applications, by implementing an ester synthesis reaction at a low reaction temperature using lipase, which is an oil hydrolysis enzyme, as a catalyst and where the materials include 12-hydroxystearic acid or derivative (mainly ester) thereof obtained by hydrogenating ricinoleic acid obtained from castor oil, and 12-hydroxy dodecanic acid or other long-chain hydroxy acid that can be synthesized from plant-based seed oil, etc. Using this method, it is now possible to obtain an excellent thermoplastic elastomer.
For your information, Fig. 1 shows the chemical formula of 12-hydroxystearic acid, while Fig. 2 shows the chemical formula of long-chain hydroxy acid. Both acids are saturated hydroxy fatty acids.

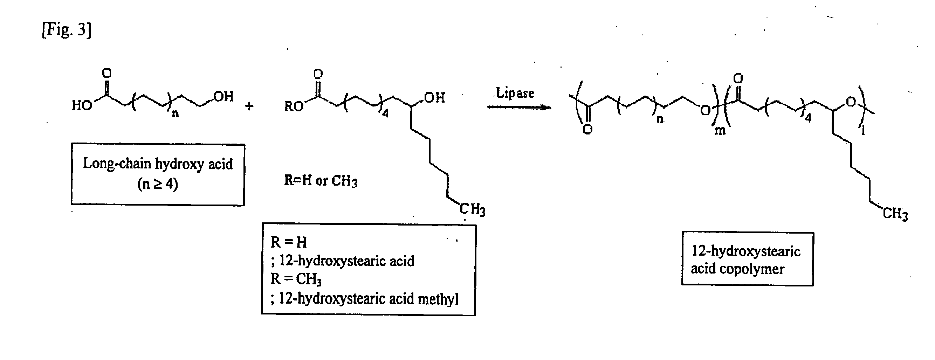

Fig. 3 shows the synthesis reaction of the target of the present invention, or specifically 12-hydroxystearic acid copolymer.

12-hydroxystearic acid or derivative thereof used as a material under the present invention has a molecular weight of approx. 300 and has a hydroxyl group at position 12 of the molecule, with carboxylic acid or carboxylic acid ester present at the end of the molecule. Accordingly, it undergoes self-condensation through esterification or transesterification, or undergoes esterification or transesterification with long-chain hydroxy acid, to form a linear macromolecule. To implement these reactions to form a macromolecule, an immobilized lipase must be used in the presence of a molecular sieve or under decompression to remove the condensate in an efficient manner.

The invention described in the present application for patent basically consists of the first through fourth inventions described below (these are hereinafter referred to as the "present invention" unless otherwise specified).
To be specific, the present invention encompasses the following:
(1) 12-hydroxystearic acid copolymer expressed by Formula 3 below, obtained by copolymerization of 12-hydroxystearic acid or derivative (mainly ester) thereof expressed by Formula 1 below and long-chain hydroxy acid of n = 4 or more expressed by Formula 2 below. 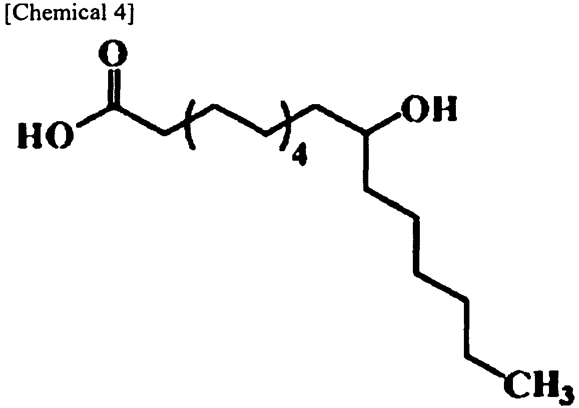 (Formula 1) 12-hydroxystearic acid 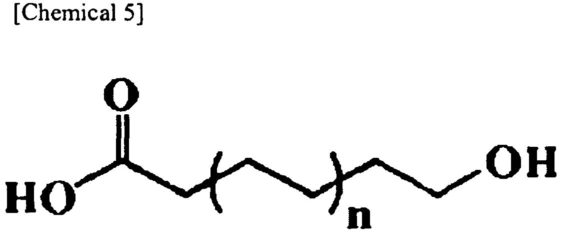 (Formula 2) Long-chain hydroxy acid 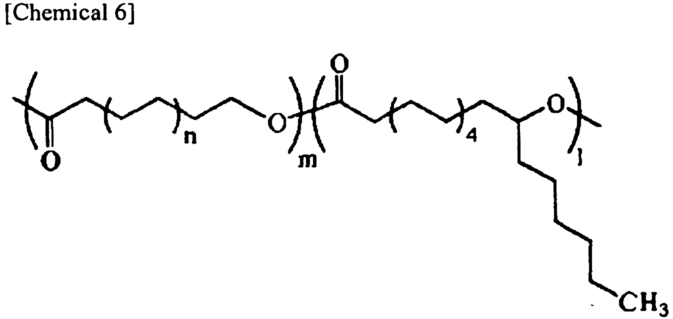 (Formula 3) 12-hydroxystearic acid copolymer
(2) 12-hydroxystearic acid copolymer according to (1) above, whose weight-average molecular weight (Mw) is 20000 or more.
(3) 12-hydroxystearic acid copolymer according to (1) or (2) above, characterized in that its content of 12-hydroxystearic acid is 15 mol% or more but less than 100 mol%.
(4) Method of manufacturing a 12-hydroxystearic acid copolymer expressed by Formula 3, characterized by synthesis from materials that include 12-hydroxystearic acid or derivative thereof expressed by Formula 1 and long-chain hydroxy acid of n = 4 or more expressed by Formula 2, by using an oil hydrolysis enzyme (lipase) as a catalyst.

Furthermore, the present invention also encompasses the following inventions that reference the aforementioned basic inventions:
(5) 12-hydroxystearic acid copolymer according to any one of (1) to (3), characterized in that its melting point by DSC is 30°C or above but below 120°C.
(6) 12-hydroxystearic acid copolymer according to any one of (1), (2), (3) and (5), characterized in that it is a thermoplastic elastomer whose hardness by Durometer-A is 30 A or above but below 90 A.

### Effects of the Invention

Based on the above, the present invention makes it possible to obtain a biodegradable copolymer offering flexibility and excellent mechanical strength, at a high yield, by means of a polymerization method using an immobilized catalyst being an oil hydrolysis enzyme (lipase), from materials that include 12-hydroxystearic acid or derivative thereof obtained by hydrogenating ricinoleic acid which in turn is obtained from castor oil, as well as long-chain hydroxy acid that can be synthesized from plant-based seed oil.
As a result, a copolymer containing 12-hydroxystearic acid of high molecular weight can be synthesized without using any harmful synthesis catalyst that contributes to environmental pollution, but by using an immobilized lipase in a reaction system accompanied by a molecular sieve under relatively mild reaction conditions. The obtained copolymer can substitute petroleum-based elastomers and achieves energy-saving, while at the same time it reduces carbon dioxide emissions substantially compared to when a thermal condensation reaction is used, consequently leading to reduced consumption of petroleum resource as well as application to medical biomaterials.

### Brief Description of the Drawings

- [Fig. 1]: Chemical formula of 12-cydroxy stearic acid
- [Fig. 2]: Chemical formula of long-chain hydroxy acid
- [Fig. 3]: Synthesis reaction formula of 12-hydroxy acid copolymer
- [Fig. 4]: 1H-NMR spectrum of 12-hydroxy acid copolymer (12HS content: 23 mol%)
- [Fig. 5]: DSC profiles (temperature fall curves) of various copolymers
- [Fig. 6]: Composition (12HS content) dependence of melting point and crystallization temperature
- [Fig. 7]: Composition (12HS content) dependence of Young's modulus
- [Fig. 8]: Composition (12HS content) dependence of hardness
- [Fig. 9]: Biodegradability of 12-hydroxy acid copolymer (12HS content: 36 mol%) (BOD test)
- [Fig. 10]: Chemical recyclability of 12-hydroxy acid copolymer (12HS content: 36 mol%)
- [Fig. 11]: Tracing chart of Fig. 4

### Best Mode for Carrying Out the Invention

The present invention is explained in detail below.
The present invention relates to a copolymer of high molecular weight offering flexibility and excellent biodegradability and thereby proving valuable in industrial applications, by subjecting 12-hydroxystearic acid or derivative thereof which is a petroleum-free material, to an enzyme reaction associated with excellent reaction efficiency at a lower reaction temperature than normal chemical reactions, as well as a method of manufacturing the same.

A 12-hydroxy acid copolymer with a weight-average molecular weight of 3000 or less can be synthesized relatively easily by means of thermal polycondensation reaction or esterification or transesterification using lipase. Because the molecular weight of such 12-hydroxy acid copolymer composition significantly affects mechanical strength, rubber-like elasticity, etc., however, the obtained copolymer, if its molecular weight is low, cannot be used to substitute thermoplastic elastomers traditionally used in industrial applications.
The 12-hydroxystearic acid or derivative thereof and long-chain hydroxy acid used under the present invention are both bifunctional compounds having carboxyl and hydroxyl groups in their molecule, and therefore the generated copolymer has a polymer structure where the two molecules are ester-bonded.
Also under the present invention, condensation water (when 12-hydroxystearic acid is used) or low-molecular-weight alcohol (when 12-hydroxystearic acid ester is used) generated in the polymerization process can be removed from the reaction system as soon as it is generated to prepare a polyester with a weight-average molecular weight of 20000 or more. This way, a polyester useful as a thermoplastic elastomer could be obtained.

The 12-hydroxystearic acid or derivative thereof from which the 12-hydroxystearic acid copolymer is produced can be synthesized by hydrogenating ricinoleic acid obtained from castor oil. In general, such 12-hydroxystearic acid or derivative thereof is used in lubricants and additives.
On the other hand, 12-hydroxy dodecanic acid and other long-chain hydroxy acids can be synthesized from plant-based seed oil, etc., and they are also found in small quantities in plant leaves, etc. They are characterized by a long main methylene chain and a polyethylene-like crystalline structure.

Under the present invention, lipase that can initiate a condensation reaction at relatively low temperatures is used to obtain a high-molecular-weight copolymer of 12-hydroxystearic acid or derivative thereof and long-chain hydroxy acid, and this not only contributes to energy-saving but it also prevents toxic catalysts from mixing into the generated polyester.
Furthermore, use of an immobilized enzyme allows for repeated use of the enzyme to implement a series of condensation reactions, and thermal stability also improves compare to when a single enzyme not chemically modified is used. As a result, the enzyme reaction field can be controlled in a stable manner with greater ease and removal from the reaction system is also easy.
In other words, a 12-hydroxy acid copolymer of high purity can be obtained easily in an energy-efficient manner. Fig. 3 shows the formula for reacting 12-hydroxystearic acid or derivative thereof (methyl ester) and long-chain hydroxy acid using an immobilized lipase as a catalyst.

The copolymer of 12-hydroxystearic acid or derivative thereof and long-chain hydroxy acid, as obtained by the present invention, has a high weight-average molecular weight and thus provides a polyester that cannot be easily achieved with normal enzyme reactions. To synthesize a polyester of high molecular weight from hydroxy acid or hydroxy acid ester using lipase that has reverse reactivity, generated water or lower alcohol must be removed as soon as it is generated. To do this, the reaction system must be controlled by maintaining it in a decompressed state, etc. In this sense, presence of molecular sieves 4A or other synthetic zeolite compound is desirable as such compound not only promotes ester synthesis reaction in a simple, easy manner, but it also increases the molecular size of polyester.

Synthetic zeolite is an inorganic porous substance having pores of a uniform size. Accordingly, molecules smaller than the pore size are adsorbed into the pores, while molecules larger than the pore size cannot enter the pores and thus are not adsorbed, which allows for separation of these two groups of molecules. In other words, synthetic zeolite has a molecule screening effect and can separate water and low-molecular-weight alcohol that generate when a copolymer composition is produced. The 12-hydroxy acid copolymer of high molecular weight thus obtained provides a non-petroleum thermoplastic elastomer that can replace traditional thermoplastic elastomers, etc., derived from petroleum materials.

In addition, the enzyme used under the present invention can be any of the commercially available lipase products derived from various fungus bodies. According to the examination by the inventor, good polyester synthesis results were achieved with an immobilized lipase derived from Candida antarctica (Novozym 435 by Novozymes Japan Ltd.). However, the applicable enzymes are not limited to those derived from this strain, and any enzymes can be used as long as they demonstrate enough activity and stability to permit synthesis, in an industrial setting, of a copolymer of 12-hydroxystearic acid and long-chain hydroxy acid with a weight-average molecular weight of 20000 or more, and also as long as their price is reasonable after considering their enzymatic performance. As for the method to immobilize such enzyme, the present invention adsorbs and immobilizes the enzyme onto an inorganic or organic carrier. However, any other method can be used such as cross-linking enzymes to obtain an immobilized enzyme insoluble in the synthesis reaction system (cross-linking method) or immobilizing the enzyme by including it into alginic acid gel, synthetic polymer gel or other polymer gel. The immobilization method is not specifically limited and any method can be used as long as it is simple and the immobilized enzyme presents high activity and stability.

As for the method to check the molecular structure and physical properties of the 12-hydroxy acid copolymer obtained by the present invention, it is desirable to evaluate/measure them using FT-IR, ¹H-NMR, ¹³C-NMR as well as tensile test, hardness measurement, etc.

### (Examples)

The present invention is explained in greater detail below using examples.

### Example 1

With respect to a copolymer containing 12-hydroxystearic acid, a copolymer composition was synthesized via enzyme reaction by changing the ratio (mol%) of 12-hydroxy dodecanic acid (12HD) to 12-hydroxystearic acid (12HS), in order to examine the yield and weight-average molecular weight (Mw) of the copolymer composition as well as polydispersity (Mw/Mn, also called molecular weight distribution index) which shows the ratio of weight-average molecular weight to number-average molecular weight (Mn), or specifically the degree of dispersion of the molecular weight of the polymer compared to its peak molecular weight. Measurement was performed using a RI detector accompanied by SEC columns (Shodex K-804L + K-800D by Showa Denko Co., Tokyo, Japan). Chloroform was introduced as a solvent at a flow rate of 1.0 mL 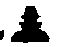 min⁻¹ to determine the molecular weight in terms of polystyrene. Reaction was implemented using a test tube with screw, where molecular sieves 4A were attached to the top of the test tube to efficiently remove the condensate. Fifty percent by weight (150 mg) of immobilized lipase (Novozym 435 by Novozymes Japan Ltd.) was used per the specified amount of matrix (total 300 mg) to cause reaction in toluene (150 ml) at 90°C. The results are shown in Table 1.
The composition ratios of copolymers were measured by ¹H-NMR. As a result, copolymers with a weight-average molecular weight of 20,000 or more were obtained at a high yield exceeding 80% at all ratios.

**[Table 1]**

| 12HD-12HS Copolymers Synthesized Using Immobilized Lipase as Catalyst | | | | | |
|---|---|---|---|---|---|
| No. | Materials Charge ratio 12HD/12HS | Reaction time h | Composition (mol%) 12HD/12HS | Yield % | Copolymer Mw (Mw/Mn) |
| 1 | 100/0 | 96 | 100/0 | 88 | 104,600 (2.9) |
| 2 | 93/7 | 96 | 93/7 | 86 | 100,400 (3.0) |
| 3 | 85/15 | 96 | 85/15 | 85 | 92,300 (2.8) |
| 4 | 77/23 | 96 | 77/23 | 83 | 98,900 (3.0) |
| 5 | 69/31 | 96 | 69/31 | 85 | 95,600 (2.8) |
| 6 | 64/36 | 96 | 64/36 | 83 | 97,500 (2.8) |
| 7 | 59/41 | 96 | 59/41 | 83 | 93,200 (3.0) |
| 8 | 40/60 | 96 | 40/60 | 85 | 98,900 (3.2) |
| 9 | 0/100 | 96 | 0/100 | 84 | 118,200 (3.3) |
| 10 | 77/23 | 20 | 79/21 | 81 | 49,600 (3.5) |

### Example 2

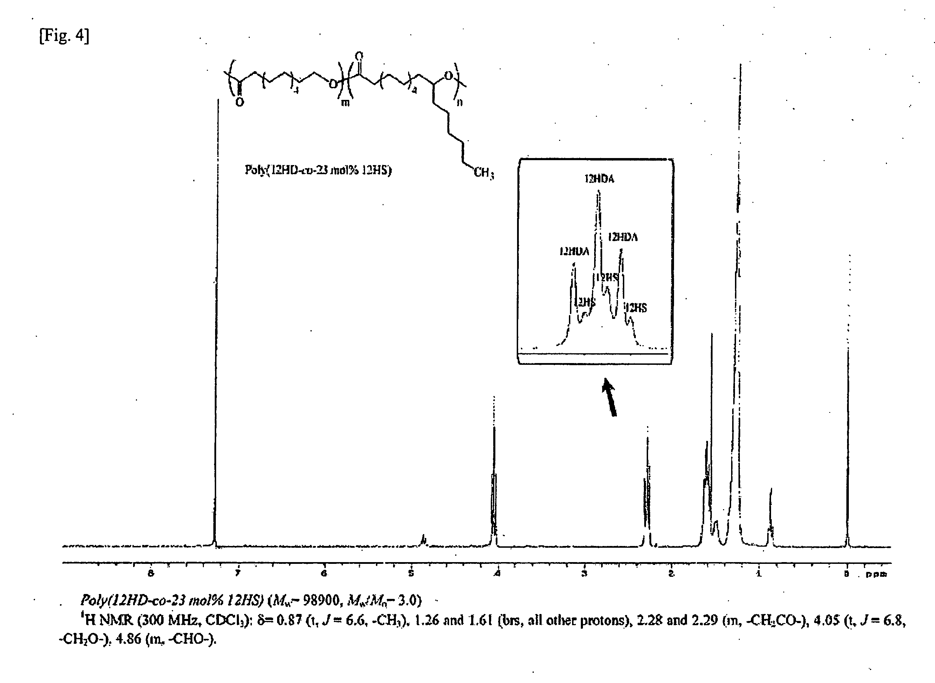

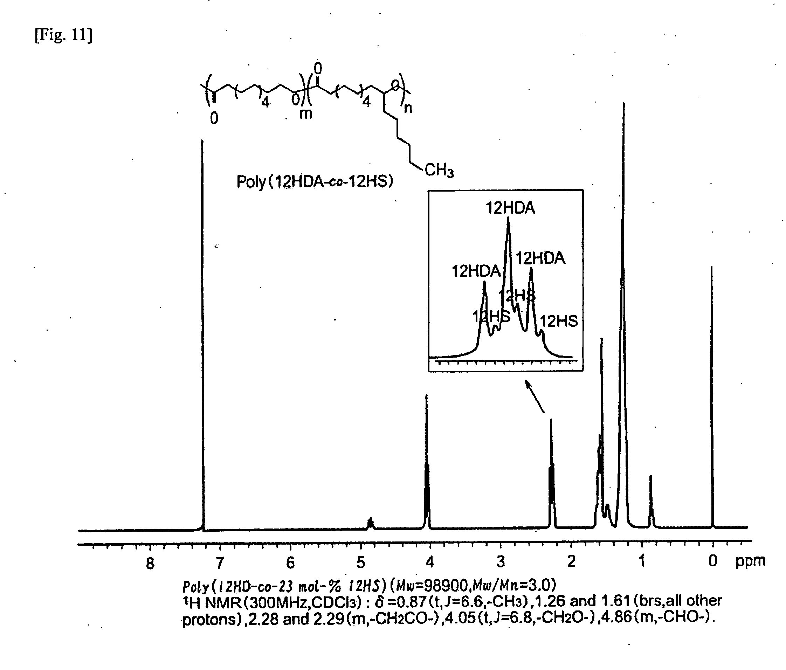

As one example of the obtained 12-hydroxy acid copolymers, ¹H-NMR structural analysis of 12-hydroxy acid copolymer No. 4 in Example 1 (weight-average molecular weight: 98,900, polydispersity (Mw/Mn): 3.0, 12HS content: 23 mol%) was conducted. The results are shown in Fig. 4 (Fig. 11 is a tracing chart of Fig. 4).
Measurement was performed on the Lambda 300 Fourier Transform Spectrometer (JEOL Ltd., Tokyo, Japan) at 300 MHz using heavy chloroform. From the spectral assignment, generation of 12-hydroxy acid copolymer was confirmed.

### Example 3

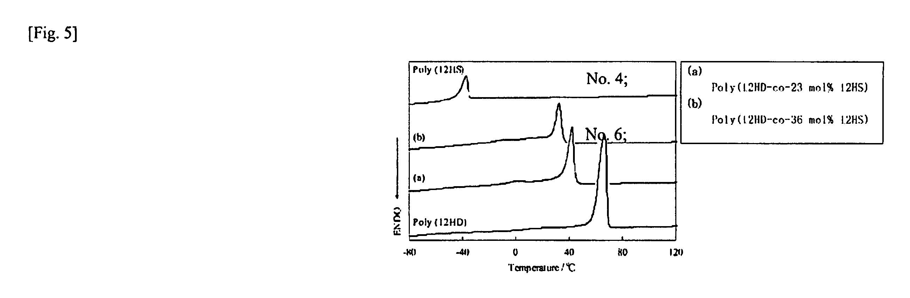

The physical properties of 12-hydroxy acid copolymers obtained by the present invention were examined. Among the thermal properties, the melting point and crystallization temperature were measured using a differential scanning calorimeter (DSC by Shimadzu, Kyoto, Japan) (rate of rise/fall in temperature: 10 °C/min). Fig. 5 shows the measured results (temperature fall curves) of two types of 12-hydroxy acid copolymers containing different amounts of 12-hydroxystearic acid (12HS), or specifically 12-hydroxy acid copolymers No. 4 and No. 6 obtained in Example 1, in comparison with two types of 12HS and 12HD homopolymers.
The two types of 12-hydroxy acid copolymers both indicated a crystallization temperature higher than that of the 12HS homopolymer but lower than that of the 12HD homopolymer. When the two types of copolymers were compared, copolymer No. 6 with the higher 12HS content indicated a lower crystallization temperature. This confirms that the thermal properties of a 12-hydroxy acid copolymer can be changed as desired according to the content of 12HS.

### Example 4

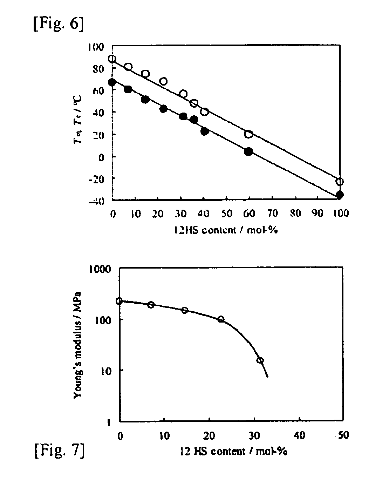

Examining the same thermal properties, Fig. 6 shows the melting points and crystallization temperatures of a 12-hydroxy acid copolymer measured at different contents of 12-hydroxystearic acid (12HS).
According to Fig. 6, both the melting point and crystallization temperature of the 12-hydroxy acid copolymer drop linearly as the 12HS content of the 12-hydroxy acid copolymer increases, indicating that the thermal properties of the copolymer can be changed as desired by changing its 12HS content.

### Example 5

Similarly, the Young's modulus of an obtained 12-hydroxy acid copolymer was measured by changing its 12-hydroxystearic acid (12HS) content. The results are shown in Fig. 7. The Young's modulus was measured on a tensile tester (Autograph by SHIMADZU Co., Tokyo, Japan) using a film sample prepared by the solvent casting method. The Young's modulus of this copolymer dropped as the 12HS content increased, and changed significantly over a range of 20 mol% or more. This confirms that the Young's modulus of the copolymer can be changed significantly according to its 12HS content.

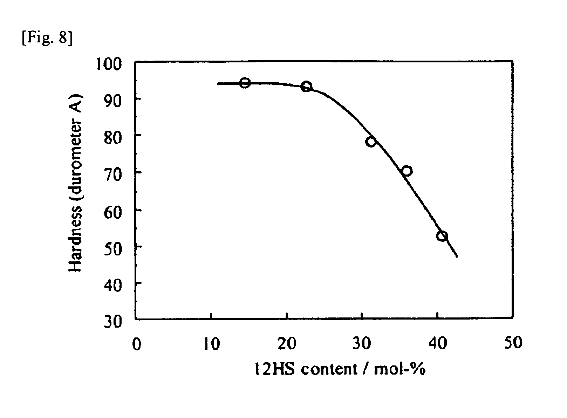

Fig. 8 shows how the Durometer A hardness of a 12-hydroxy acid copolymer changes when its 12HS content is changed. Hardness was measured in conformance with ASTM D 2240 using a 2-mm thick pressed sheet prepared by a thermal press. According to Fig. 8, the hardness of this copolymer changed significantly over a 12HS content range of 15 mol% or more, as was the case with the Young's modulus, and this confirms that the hardness of the copolymer can be changed significantly according to its 12HS content.

### Example 6

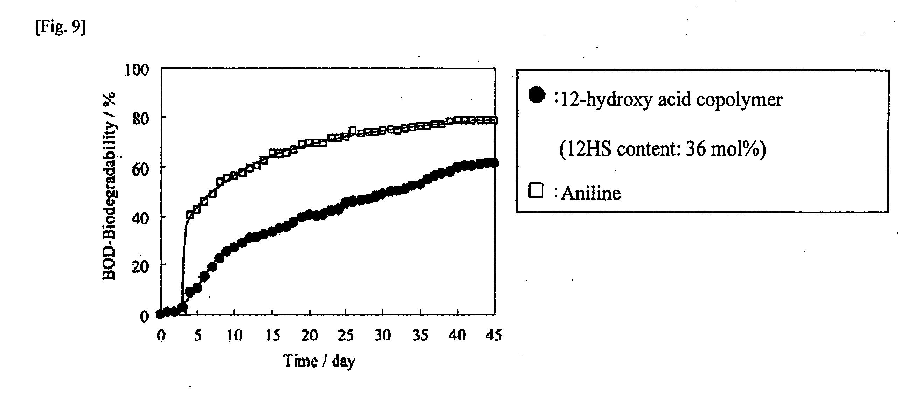

Fig. 9 shows the results of examining the biodegradability of a 12-hydroxy acid copolymer. To be specific, Fig. 9 shows the biodegradability of a copolymer containing 12-hydroxystearic acid (12HS) by 36 mol% as measured by the BOD method (OECD Guidelines for Testing of Chemicals, 301C, Modified MITI Test, Organization for Economic Cooperation and Development (OECD), Paris, 1981) using aniline as a biodegradability index.
Compared to aniline having good biodegradability, the copolymer began degrading roughly at the same time as aniline and its subsequent degradation rate also remained at a level only slightly lower than aniline.
These results clearly show that the 12-hydroxy acid copolymer has excellent biodegradability (approx. 60% degrades in 40 days).

### Example 7

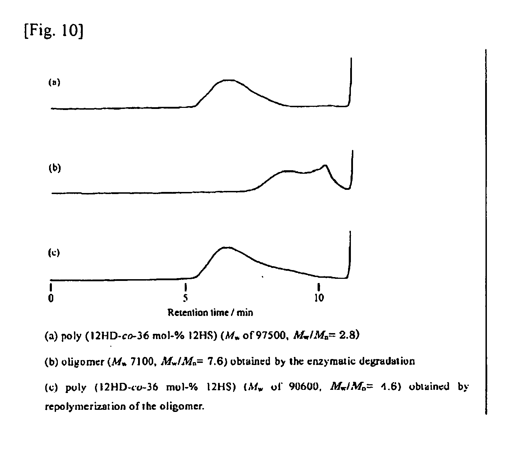

To examine the chemical recyclability of a 12-hydroxy acid copolymer obtained by the present invention, profile changes were measured with a size exclusion chromatography (SEC) for (a) poly(12HD-co-36 mol% 12HS), (b) oligomer produced via enzymatic hydrolysis of poly(12HD-co-36 mol% 12HS), and (c) poly(12HD-co-36 mol% 12HS) obtained by repolymerizing this oligomer. The results are shown in Fig. 10. Compared to that of the polymer (a), the oligomer (b) had a longer holding time. This indicates that the oligomer had a smaller molecular weight than the polymer, meaning that the oligomer had degraded to roughly the same molecular weight. Since the polymer (c) obtained by repolymerizing this oligomer had the same holding time as (a), it is clear that the repolymerization of the oligomer produced the same polymer as (a).
Based on the above, there is a reversible relationship of degradation and polymerization between this 12-hydroxy acid copolymer and the oligomer which is a degradation product thereof. In other words, this copolymer can be degraded and recovered as an oligomer, which can be then polymerized again to synthesize the copolymer.
In other words, this polymer is considered to have excellent chemical recyclability, which is growing in importance from the viewpoint of effective utilization of petroleum resource, and when an immobilized lipase is used, it can be produced without polluting the environment.

From the examples explained above, it is possible to change the thermal properties and mechanical properties of a 12-hydroxy acid copolymer composition over practical ranges by changing the content ratio of copolymerized 12-hydroxystearic acid and long-chain hydroxy acid to form a thermoplastic elastomer with a molecular weight of 20000 or more and having a different composition, and the obtained thermoplastic elastomer also offers biodegradability and chemical recyclability.

## Claims

1. 2-hydroxystearic acid copolymer expressed by Formula 3 below 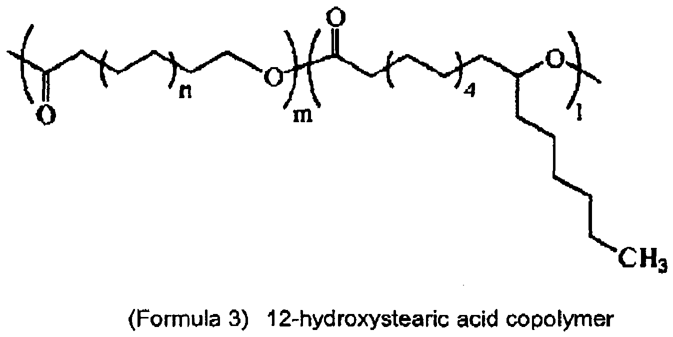 obtained by copolymerization of 12-hydroxystearic acid expressed by Formula 1 below 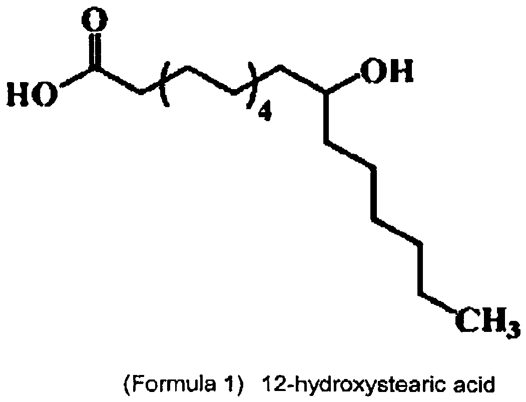 or its ester and long-chain hydroxy acid of n = 4 or more expressed by Formula 2 below.

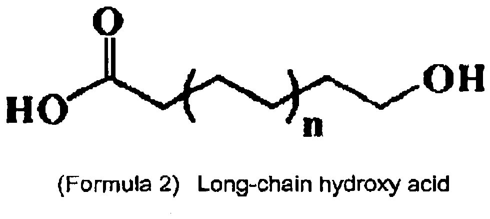

2. 12-hydroxystearic acid copolymer according to Claim 1, whose weight-average molecular weight (Mw) is 20000 or more.

3. 12-hydroxystearic acid copolymer according to Claim 1 or 2, **characterized in that** its content of 12-hydroxystearic acid is 15 mol% or more but less than 100 mol%.

4. Method of manufacturing a 12-hydroxystearic acid copolymer expressed by Formula 3 below, 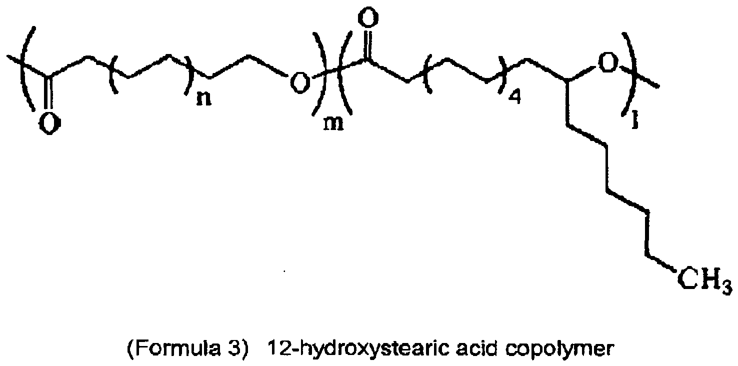 **characterized by** synthesis from materials that include 12-hydroxystearic acid expressed by Formula 1 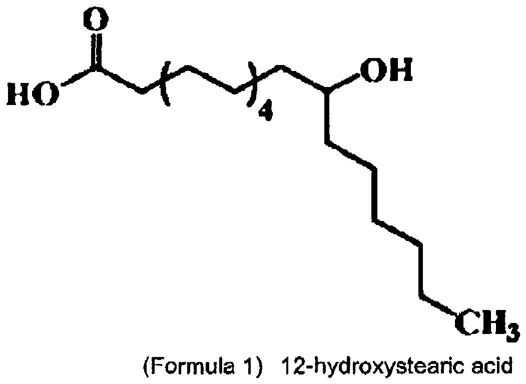 or its ester and long-chain hydroxy acid of n = 4 or more expressed by Formula 2, 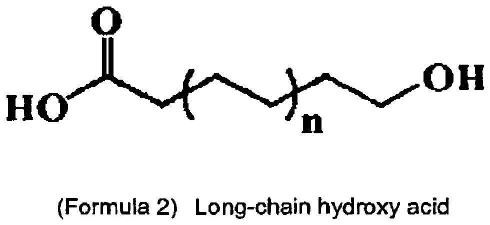 by using an oil hydrolysis enzyme (lipase) as a catalyst.

5. 12-hydroxystearic acid copolymer according to any one of Claims 1 to 3, **characterized in that** its melting point as measured by DSC where a rate of rise/fall in temperature is 10 °C/min is 30°C or above but below 120°C.

6. 12-hydroxystearic acid copolymer according to any one of Claims 1, 2, 3 and 5. **characterized in that** it is a thermoplastic elastomer whose hardness as measured by Durometer-A in conformance with ASTM D 2240 using a 2-mm thick pressed sheet prepared by a thermal press is 30 A or above but below 90 A.

## Patentansprüche

1. 12-Hydroxystearinsäurecopolymer, ausgedrückt durch die Formel 3 unten 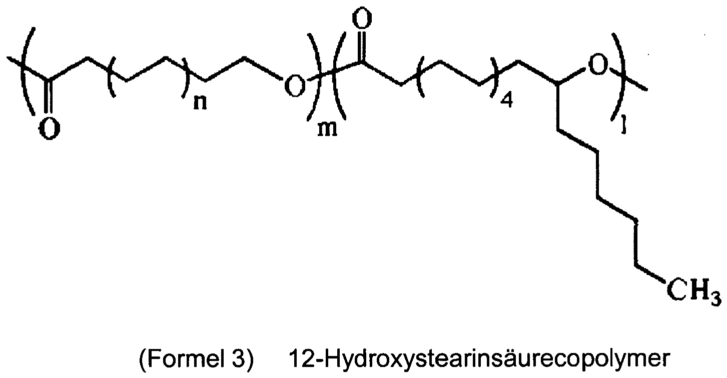 erhalten durch Copolymerisation von 12-Hydroxystearinsäure, ausgedrückt durch die Formel 1 unten 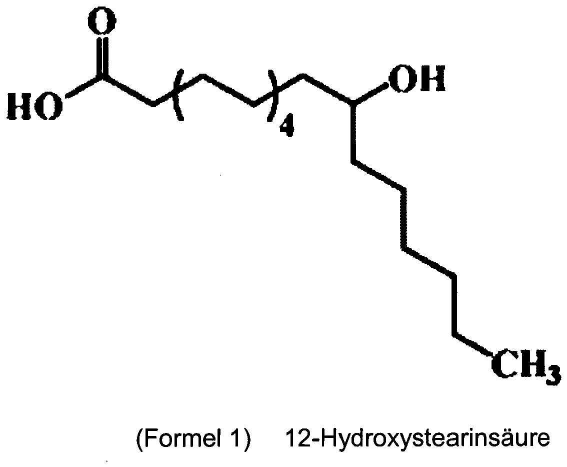 oder deren Ester und eine langkettige Hydroxysäure mit n = 4 oder mehr, ausgedrückt durch die Formel 2 unten.

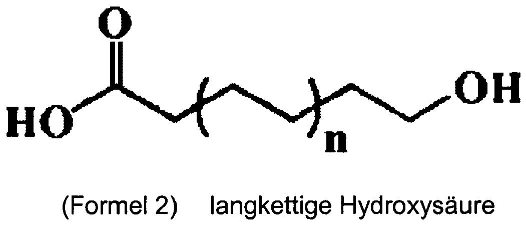

2. 12-Hydroxystearinsäurecopolymer gemäß Anspruch 1, dessen gewichtsmittleres Molekulargewicht (Mw) 20000 oder mehr ist.

3. 12-Hydroxystearinsäurecopolymer gemäß Anspruch 1 oder 2, dadurch charakterisiert, dass dessen Gehalt an 12-Hydroxystearinsäure 15 Mol-% oder mehr ist, jedoch weniger als 100 Mol-%.

4. Verfahren zum Herstellen eines 12-Hydroxystearinsäurecopolymers, ausgedrückt durch die Formel 3 unten 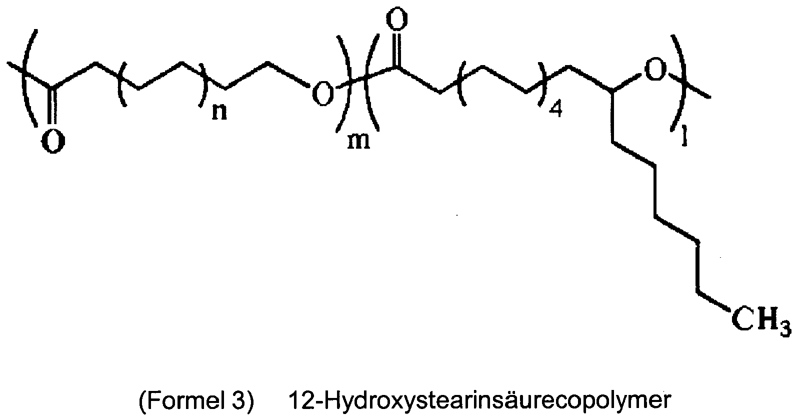 charakterisiert durch die Synthese aus Materialien, die 12-Hydroxystearinsäure, die ausgedrückt ist durch Formel 1 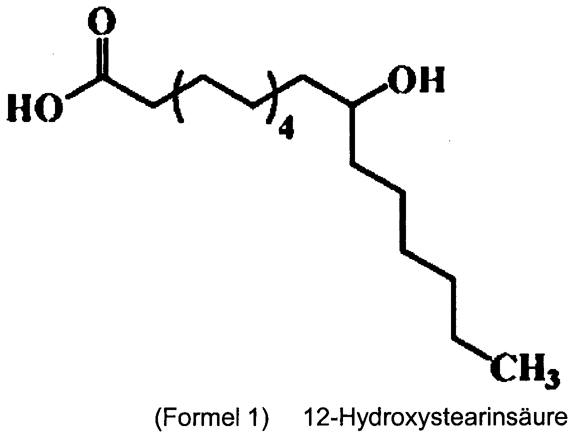 oder deren Ester und eine langkettige Hydroxysäure mit n = 4 oder mehr ist, ausgedrückt durch die Formel 2, 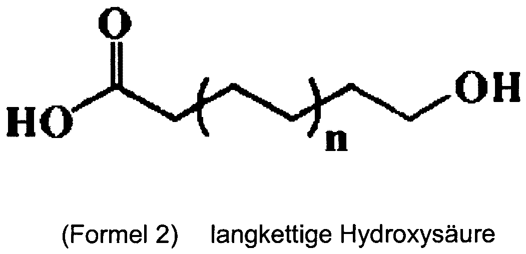 einschließen,
unter Verwendung eines Ölhydrolyseenzyms (Lipase) als Katalysator.

5. 12-Hydroxystearinsäurecopolymer gemäß einem der Ansprüche 1 bis 3, dadurch charakterisiert, dass der Schmelzpunkt bei 30°C oder oberhalb, jedoch unterhalb von 120°C liegt, wie er durch dynamische Differenzkalorimetrie gemessen wird, wobei eine Geschwindigkeit des Ansteigens/Abfallens der Temperatur bei 10 °C/min liegt.

6. 12-Hydroxystearinsäurecopolymer gemäß einem der Ansprüche 1, 2, 3 und 5, dadurch charakterisiert, dass es ein thermoplastisches Elastomer ist, dessen Härte 30 A oder mehr, jedoch weniger als 90 A beträgt, ermittelt durch eine Durometer-A-Messung in Übereinstimmung mit ASTM D 2240 unter Verwendung einer 2 mm dicken gepressten Lage, die durch eine thermische Presse hergestellt wurde.

## Revendications

1. Copolymère d'acide 12 hydroxy stéarique répondant à la formule 3 ci-dessous 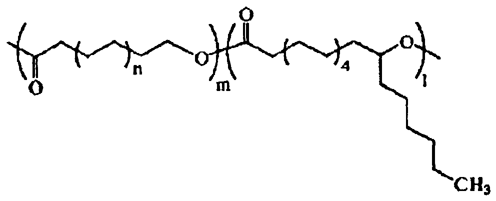 (formule 3) - copolymère d'acide 12 hydroxy stéarique
obtenu par copolymérisation d'un acide 12 hydroxy stéarique répondant à la formule 1 ci-dessous 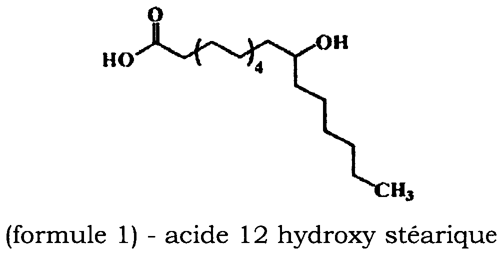 ou d'un ester de cet acide et d'un hydroxyl acide à longue chaine dans lequel n = 4 ou plus répondant à la formule 2 ci-dessous

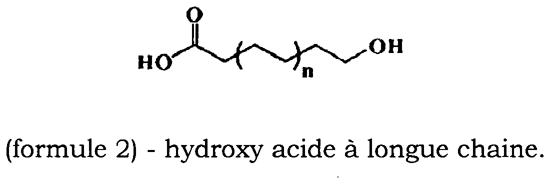

2. Copolymère d'acide 12 hydroxy stéarique conforme à la revendication 1, dont le poids moléculaire moyen (Mw) est de 20000 ou plus.

3. Copolymère d'acide 12 hydroxy stéarique conforme à la revendication 1 ou 2,
**caractérisé en ce que**
sa teneur en acide 12 hydroxy stéarique est de 15 % en moles ou plus mais est inférieure à 100 % en moles.

4. Procédé d'obtention d'un copolymère d'acide 12 hydroxy stéarique répondant à la formule 3 ci-dessous 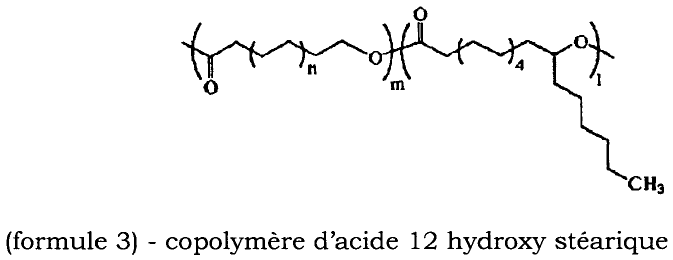 **caractérisé en ce qu'**
il est synthétisé à partir de matériaux renfermant un acide 12 hydroxy stéarique répondant à la formule 1, 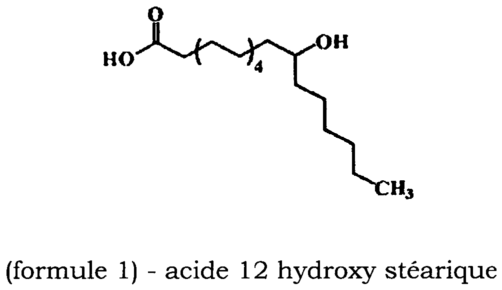 ou l'un de ses esters et un hydroxy acide à longue chaine dans lequel n = 4 ou plus répondant à la formule 2 ci-dessous 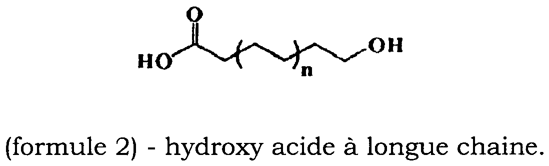 en utilisant en tant que catalyseur une enzyme d'hydrolyse des lipides (lipase).

5. Copolymère d'acide 12 hydroxy stéarique conforme à l'une quelconque des revendications 1 à 3,
**caractérisé en ce que**
son point de fusion mesuré par DSC avec un taux d'augmentation/ chute de température de 10°C/min est 30°C ou plus mais est inférieur à 120°C.

6. Copolymère d'acide 12 hydroxy stéarique conforme à l'une quelconque des revendications 1, 2, 3 et 5,
**caractérisé en ce qu'**
il correspond à un élastomère thermoplastique dont la dureté mesurée par un duromètre A conformément à la norme ASTM D2240 en utilisant une feuille de pression de deux millimètres d'épaisseur préparée par compression thermique est de 30 A ou plus mais est inférieure à 90 A.
